# EUROPÄISCHE PATENTSCHRIFT

(11) **EP 1 306 107 B1**
(45) Veröffentlichungstag und Bekanntmachung des Hinweises auf die Patenterteilung: **01.06.2005**
(21) Anmeldenummer: 02022556.1
(22) Anmeldetag: 08.10.2002
(51) Int. Cl.: A61N 5/06

(54) **Rechteckige Rahmenanordnung mit ein bis zwei scheibenförmigen Strahlungsfiltern und Bräunungsmodul**
Rectangular frame arrangement with one or two disc-shaped radiation filters and tanning module
Châssis rectangulaire avec un ou deux filtres de rayonnement en forme de disque et module de bronzage

(30) Priorität: 24.10.2001 DE 10151841
(43) Veröffentlichungstag der Anmeldung: 02.05.2003
(73) Patentinhaber: Heraeus Noblelight GmbH, 63450 Hanau (DE)
(72) Erfinder: Ullrich, Bernd, 63526 Erlensee (DE); Berger, Ulrich, 63599 Biebergemünd (DE)
(74) Vertreter: Kühn, Hans-Christian

(56) Entgegenhaltungen:
- DE-U- 29 705 097

## Beschreibung

Die Erfindung betrifft eine rechteckige Rahmenanordnung mit ein bis zwei scheibenförmigen Strahlungsfiltern zum Abfiltern eines Spektrums eines Bräunungsstrahler sowie ein Bräunungsmodul mit einer solchen Rahmenanordnung.

Der Einsatz von scheibenförmigen Strahlungsfiltem in Bräunungsgeräten ist bekannt. So offenbart beispielsweise die DE 29 41 467 A1 ein Bräunungsmodul mit rechteckigem Gehäuse inklusive eines Wärmefilters in einer Gehäusewand. Im Gehäuse ist ein Reflektor angeordnet, in welchen sich ein UV-Filter zwischen dem Bräunungsstrahler und dem Wärmefilter befindet.

Die DE 195 16 603 A1 offenbart ein Niederdruckgesichtsfeld für Bräunungsgeräte, wobei ein rechteckiges Gehäuse inklusive Reflektor und Filterscheibe zum Einsatz kommt. Das Gehäuse ist zum Einbau mehrerer UVC-Röhren geeignet. Die Seite der Filterscheibe, die den Röhren zugewandt ist, ist mit einer Schicht aus UV-Leuchtpigmenten beschichtet.

Die DE 36 31 427 C2 beschreibt ein Bestrahlungsgerät mit einem rechteckigen Gehäuse, einem Reflektor sowie einer Filterscheibe. Zur Sicherung der Filterscheibe gegen Bruch ist ein Druckschalter vorgesehen, der von der Filterscheibe in seiner eingedrückten Stellung gehalten wird, jedoch bei Bruch der Filterscheibe aus dieser Stellung heraustritt und die Strahlungsquelle abschaltet. Die Filterscheibe ist im Gehäuse teilweise mit einer Klebschicht befestigt.

Die DE 39 27 695 C2 offenbart ein Bräunungsgerät mit einem schwenkbar angeordneten Interferenzfilter. In Strahlenaustrittsrichtung ist dem Interferenzfilter ein Infrarotfilter nachgeordnet. Je nach Neigung des Interferenzfilters im Strahlenaustritt wird die Grenze des Transmissionsspektrums zum kurzwelligeren UV-B-Anteil oder zum langwelligeren UV-A-Anteil verschoben.

Damit ist durch das Verschwenken des Filters das Strahlungsspektrum auf den Hauttyp der zu bestrahlenden Person einstellbar.

Die DE 40 37 483 C2 beschreibt ein UV-Bestrahlungsgerät mit Bruchsicherung für eine Filterglasscheibe, wobei an deren Umfang eine stromdurchflossene elektrische Leiterbahn angeordnet ist. Bei Bruch der Filterglasscheibe wird die Leiterbahn und damit der Strom unterbrochen und der Bräunungsstrahler abgeschaltet.

Die DE 297 05 097 U offenbart eine rechteckige Rahmenanordnung mit einem scheibenförmigen Strahlungsfilter, wobei die Rahmenanordnung vier Ecken und vier Seitenkanten aufweist und an einer ersten Seitenkante mindestens ein erstes Federprofil (14) und an einer der ersten Seitenkante gegenüberliegenden zweiten Seitenkante mindestens ein zweites Federprofil (14) aufweist, wobei zumindest das erste Federprofil (14) U-förmig mit einem ersten Boden, einer ersten oberen Seitenflanke und einer ersten unteren Seitenflanke ausgestaltet ist.

Es stellt sich das Problem, eine rechteckige Rahmenanordnung mit ein bis zwei scheibenförmigen Strahlungsfiltem zur Verfügung zu stellen, die einen einfachen Ein- und Ausbau beziehungsweise Wechsel der Strahlungsfilter erlaubt. Die Rahmenanordnung soll in einfacher Art und Weise in einem Bräunungsmodul eingesetzt werden können.

Das Problem wird durch eine rechteckige Rahmenanordnung gelöst, wobei die Rahmenanordnung vier Ecken und vier Seitenkanten aufweist und an einer ersten Seitenkante mindestens ein erstes Federprofil und an einer der ersten Seitenkante gegenüberliegenden zweiten Seitenkante mindestens ein zweites Federprofil aufweist, wobei das erste und das zweite Federprofil durch mindestens einen Steg miteinander verbunden sind und zumindest das erste Federprofil U-förmig mit einem ersten Boden, einer ersten oberen Seitenflanke und einer ersten unteren Seitenflanke ausgestaltet ist, wobei ein erster Strahlungsfilter in einem ersten Randbereich zwischen dem mindestens einen Steg und der ersten unteren Seitenflanke angeordnet ist.

Eine solche Anordnung ermöglicht einen schnellen und werkzeugfreien Ein- und Ausbau der Strahlungsfilter, da die Strahlungsfilter lediglich durch die Federprofile geklemmt jedoch nicht geklebt oder verschraubt sind. Die Rahmenanordnung kann auf einfache Weise in ein Bräunungsmodul integriert werden.

Das zweite Federprofil ist vorzugsweise ebenfalls U-förmig mit einem zweiten Boden, einer zweiten oberen Seitenflanke und einer zweiten unteren Seitenflanke ausgestaltet.

Dabei ist es zur Erzeugung der Klemmkraft von Vorteil, wenn die Winkel zwischen erstem Boden und erster oberer Seitenflanke sowie zwischen erstem Boden und erster unterer Seitenflanke kleiner 90° sind. Das gilt auch für die Winkel zwischen zweitem Boden und zweiter oberer Seitenflanke sowie zwischen zweitem Boden und zweiter unterer Seitenflanke.

Allerdings ist die Erzeugung der Klemmkraft auch dadurch möglich, dass die Winkel zwischen erstem Boden und erster oberer Seitenflanke sowie zwischen erstem Boden und erster unterer Seitenflanke gleich 90° sind, dass die erste obere und die erste untere Seitenflanke jeweils mindestens einen Schlitz aufweisen und dass jeweils mindestens ein Bereich der ersten oberen und der ersten unteren Seitenflanke im Bereich der Schlitze im Hinblick auf die jeweilige Seitenflanke angewinkelt ist.
Ebenso können die Winkel zwischen zweitem Boden und zweiter oberer Seitenflanke sowie zwischen zweitem Boden und zweiter unterer Seitenflanke gleich 90° sein, wobei die zweite obere und die zweite untere Seitenflanke jeweils mindestens einen Schlitz aufweist und jeweils mindestens ein Bereich der zweiten oberen und der zweiten unteren Seitenflanke im Bereich der Schlitze im Hinblick auf die jeweilige Seitenflanke angewinkelt ist.
Dabei ist jedoch darauf zu achten, dass sich ein Strahlungsfilter leicht in die Federprofile einschieben lassen muss.

Für die Verwendung unterschiedlich langer Strahlungsfilter ist es von Vorteil, wenn der zweite Boden durch eine Stufe in einen oberen und einen unteren zweiten Boden geteilt ist. Über die Stufe lässt sich der Längenunterschied der Strahlungsfilter in einfacher Weise ausgleichen.

Dabei ist es von Vorteil, wenn der obere zweite Boden und der untere zweite Boden ungleich breit sind. So kann auch der Steg zur Verbindung des ersten mit dem zweiten Federprofil vom breiteren zweiten Bodenteil umfasst werden.

Der erste Strahlungsfilter ist vorzugsweise in einem zweiten Randbereich zwischen dem mindestens einen Steg und der zweiten unteren Seitenflanke angeordnet.

Ein zweiter Strahlungsfilter wird vorzugsweise in einem ersten Randbereich zwischen dem mindestens einen Steg und der ersten oberen Seitenflanke und in einem zweiten Randbereich zwischen dem mindestens einen Steg und der zweiten oberen Seitenflanke angeordnet.

Es hat sich bewährt, wenn die Federprofile aus Federblech gebildet sind, da es sich bei Federblech um ein kostengünstiges und einfach zu verarbeitendes Material handelt.

Insbesondere eine Rahmenanordnung mit zwei ersten Federprofilen an der ersten Seitenkante und zwei zweiten Federprofilen an der zweiten Seitenkante ist bevorzugt, wobei jeweils ein Steg eines der zwei ersten Federprofile mit einem der zwei zweiten Federprofile verbindet, und wobei die insgesamt vier Federprofile jeweils nahe einer der vier Ecken der Rahmenanordnung angeordnet sind und die Stege über mindestens eine Verbindungsstange miteinander verbunden sind.

Eine besonders einfache Fixierung ist möglich, wenn der erste Strahlungsfilter einen rechteckigen Umfang aufweist. Dabei ist der erste Strahlungsfilter vorzugsweise ein Interferenzfilter. Bevorzugt ist ein erster Strahlungsfilter mit einer Breite und einer Länge im Bereich von 210mm bis 300mm. Vorzugsweise weist der erste Strahlungsfilter eine Breite von 220mm und eine Länge von 290mm auf.

Der zweite Strahlungsfilter ist idealerweise ein UV-Filter oder ein Infrarotfilter und vorzugsweise rechteckig ausgebildet. Bevorzugt ist ein zweiter Strahlungsfilter in einer Breite und einer Länge im Bereich von 210mm bis 290mm. Insbesondere weist der zweite Strahlungsfilter eine Breite von 220mm und eine Länge von 278mm auf.

Es hat sich bewährt, wenn der Steg auf halber Höhe zwischen der ersten oberen und der ersten unteren Seitenflanke mit dem ersten Boden verbunden ist.
Außerdem ist es von Vorteil, wenn der Steg auf halber Höhe zwischen der zweiten oberen und der zweiten unteren Seitenflanke mit dem zweiten Boden verbunden ist.

Im Fall, dass das zweite Federprofil eine Stufe aufweist, ist der Steg vorzugsweise zwischen der zweiten oberen Seitenflanke und der Stufe mit dem oberen zweiten Boden verbunden.
Der Steg kann aber auch zwischen der zweiten unteren Seitenflanke und der Stufe mit dem unteren zweiten Boden verbunden sein.

Die oberen und/oder die unteren Seitenflanken weisen idealerweise einen Knick auf. Durch einen solchen Knick werden die Seitenflanken an ihrem dem jeweiligen Boden abgewandten Ende vorzugsweise so abgewinkelt, dass die Enden zumindest teilweise nicht auf einem Strahlungsfilter aufliegen, sondern von diesem etwas abstehen. Dadurch wird das Einschieben eines Strahlungsfilters in die Federprofile erleichtert, da ein Strahlungsfilter sich an dem Knick weniger verhaken kann als an dem im Vergleich dazu rauen Ende einer Seitenflanke.

Zur Sicherung des ersten Strahlungsfilters in der Rahmenanordnung ist an den Seitenkanten der Rahmenanordnung, an denen kein Federprofil vorhanden ist, vorzugsweise jeweils eine Rutschsicherung für den ersten Strahlungsfilter angeordnet. Ebenso ist an den Seitenkanten der Rahmenanordnung, an denen kein Federprofil vorhanden ist, jeweils eine Rutschsicherung für den zweiten Strahlungsfilter vorteilhaft.

Der erste Strahlungsfilter kann auf seiner dem zweiten Strahlungsfilter abgewandten Seite einen Aufdruck oder Aufkleber aufweisen. Dabei ist es insbesondere von Vorteil, wenn der Aufdruck oder Aufkleber einen undurchsichtigen Randbereich aufweist, der die Haltekonstruktion der Strahlungsfilter vor dem Auge eines Benutzers verdeckt.

Das Problem wird weiterhin gelöst durch ein Bräunungsmodul mit einem Gehäuse, einem im oder am Gehäuse angeordneten, dreidimensionalen Reflektor sowie einer oben beschriebenen rechteckigen Rahmenanordnung an einer Seite des Gehäuses, wobei der erste Strahlungsfilter die Strahlungsaustrittsfläche des Reflektors überdeckt, wobei die obere erste Seitenflanke und die obere zweite Seitenflanke des ersten Federprofils dem Reflektor zugewandt sind, während die untere erste Seitenflanke und die untere zweite Seitenflanke des zweiten Federprofils vom Reflektor abgewandt sind.

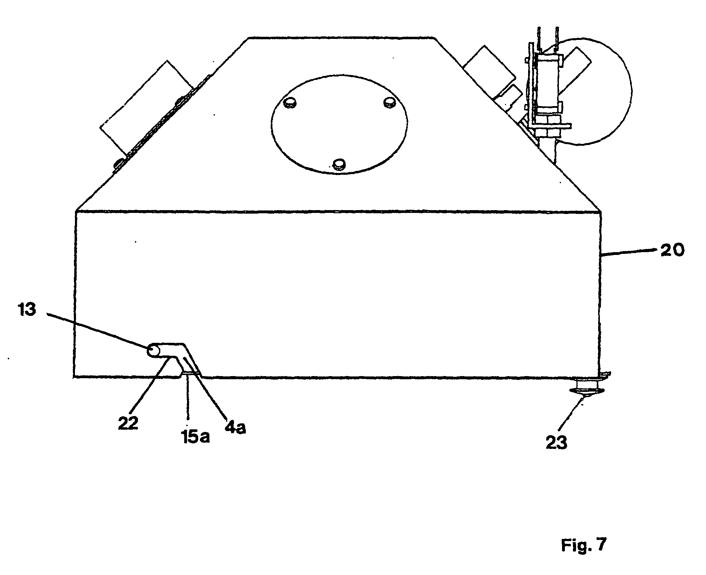

Dabei ist die rechteckige Rahmenanordnung vorzugsweise über einen Schwenkmechanismus vom Gehäuse lösbar und damit austauschbar. Dabei soll der Schwenkmechanismus ein Verkippen des Strahlungsfilters im Hinblick auf das Gehäuse ermöglichen, wobei das Lösen des Strahlungsfilters vom Gehäuse erst nach einer Verschiebung des verkippten Strahlungsfilters im Gehäuse möglich sein soll. Dadurch wird ein benutzerfreundlicher Austausch des Strahlungsfilters möglich und auch ein plötzliches Herabfallen des Strahlungsfilters vermieden, da über einen solchen Schwenkmechanismus ein Herabfallen des Strahlungsfilters und damit ein Bruch wirkungsvoll verhindert werden kann.
Die rechteckige Rahmenanordnung wird dabei idealerweise in das Gehäuse eingehakt. Insbesondere eine Öffnung gemäß Figur 7 im Gehäuse ist geeignet, die erfindungsgemäße Rahmenanordnung derart einzuhaken.
Die rechteckige Rahmenanordnung wird vorzugsweise mittels eines Schnappmechanismus in der Position fixiert.

Ein Umfang des Reflektors parallel zur Strahlungsaustrittsfläche beschreibt vorzugsweise einen Kreis, eine Ellipse, ein Rechteck oder ein Vieleck. Besonders bevorzugt ist dabei, wenn der Reflektor aus Facetten gebildet ist und der Umfang des Reflektors parallel zur Strahlungsaustrittsfläche ein Vieleck mit zwölf Ecken beschreibt.

Es hat sich bewährt, wenn der Reflektor eine Höhe von 90mm bis 95mm, insbesondere von 93,6mm, und das Zwölfeck in der Ebene der Strahlungsaustrittsfläche einen maximalen Durchmesser ( von Ecke zu Ecke ) im Bereich von 210mm bis 230mm, insbesondere von 210mm, aufweist.

Weiterhin hat es sich bewährt, wenn der Reflektor eine Höhe im Bereich von 110mm bis 125mm, insbesondere von 118,7mm, und das Zwölfeck in der Ebene der Strahlungsaustrittsfläche einen maximalen Durchmesser ( von Ecke zu Ecke ) im Bereich von 170mm bis 200mm, insbesondere von 184mm, aufweist.

Außerdem hat sich ein Reflektor bewährt, der eine Höhe im Bereich von 75mm bis 90mm, insbesondere von 83,3mm aufweist und bei dem das Zwölfeck in der Ebene der Strahlungsaustrittsfläche einen maximalen Durchmesser ( von Ecke zu Ecke ) im Bereich von 205mm bis 235mm, insbesondere von 220mm, aufweist.

Die Figurendarstellungen 1 bis 8 sollen die erfindungsgemäße Rahmenanordnung sowie das Bräunungsmodul beispielhaft erläutern. Dabei zeigt
- Figur 1: eine rechteckige Rahmenanordnung in dreidimensionaler Ansicht,
- Figur 2: die Rahmenanordnung aus Figur 1 mit allen verdeckten Linien,
- Figur 3a: ein erstes Federprofil,
- Figur 3b: ein zweites Federprofil,
- Figur 4a: ein weiteres erstes Federprofil,
- Figur 4b: ein weiteres zweites Federprofil,
- Figur 5: einen Aufdruck mit undurchsichtigem Randbereich,
- Figur 6: drei Bräunungsmodule im Schnitt,
- Figur 7: einen Schwenkmechanismus für die Rahmenanordnung,
- Figur 8: eine dreidimensionale Darstellung von drei Bräunungsmodulen.

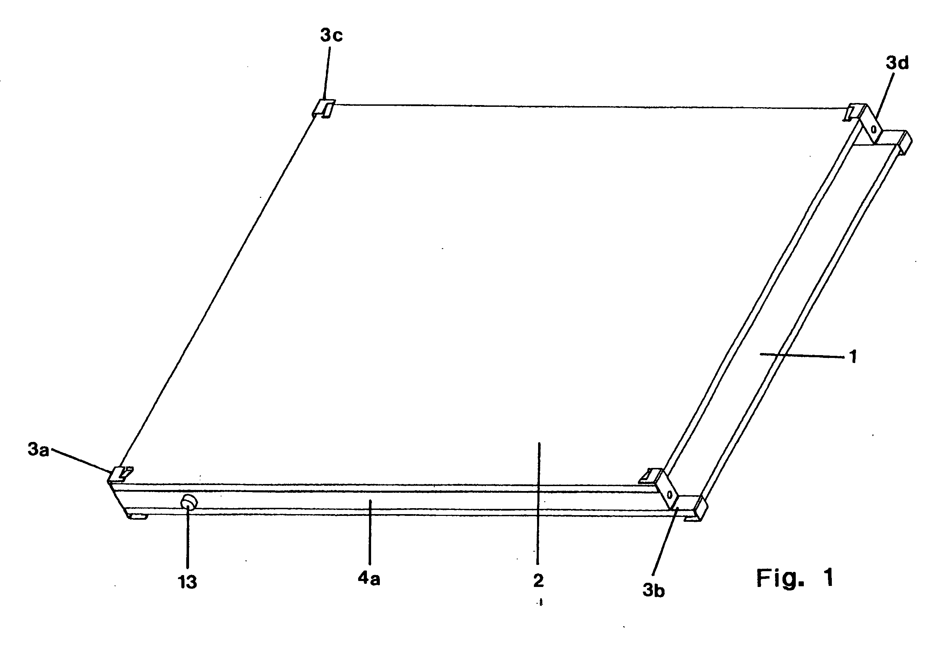

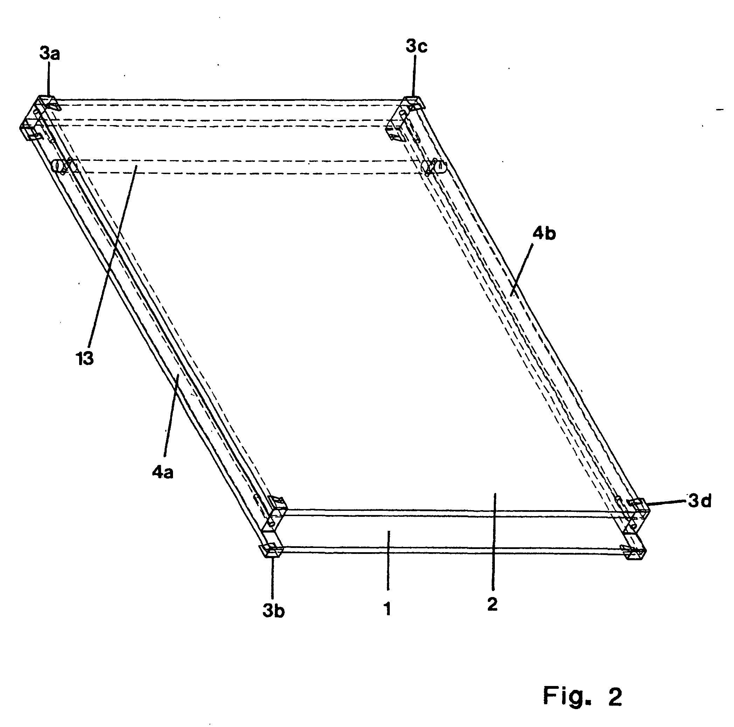

So zeigt Figur 1 eine rechteckige Rahmenanordnung in dreidimensionaler Ansicht, wobei ein erster Strahlungsfilter 1 und ein zweiter Strahlungsfilter 2 vorhanden sind. Es ist hinzuzufügen dass auch nur ein erster Strahlungsfilter 1 vorhanden sein könnte. Zwei erste Federprofile 3a, 3c befinden sich an einer Seitenkante der Strahlungsfilter und sind über Stege 4a, 4b ( siehe auch Figur 2 ) mit jeweils einem zweiten Federprofil 3b, 3d verbunden. Die beiden Stege 4a, 4b sind über eine Verbindungsstange 13 parallel zueinander miteinander verbunden. Die Verbindungsstange 13 ragt an beiden Seiten über die Stege 4a, 4b hinaus und ist daher dazu geeignet, die Rahmenanordnung in das Gehäuse eines Bräunungsmoduls einzuhaken.

Figur 2 zeigt die Rahmenanordnung aus Figur 1 mit allen verdeckten Linien.

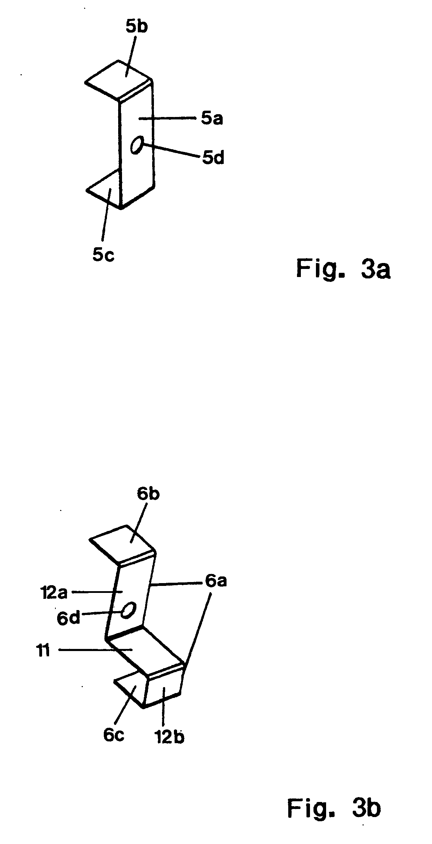

Figur 3a zeigt ein einfaches erstes Federprofil mit einem ersten Boden 5a, einer ersten oberen Seitenflanke 5b und einer ersten unteren Seitenflanke 5c. Im ersten Boden 5a befindet sich eine Öffnung 5d, über welche das erste Federprofil mit einem Steg 4a, 4b verbunden werden kann.

Figur 3b zeigt ein einfaches zweites Federprofil mit einem zweiten Boden 6a, einer zweiten oberen Seitenflanke 6b und einer zweiten unteren Seitenflanke 6c. Im zweiten Boden 6a befindet sich eine Öffnung 6d, über welche das zweite Federprofil mit einem Steg 4a, 4b verbunden werden kann. Der zweite Boden 6a ist durch eine Stufe 11 in einen oberen zweiten Boden 12a und einen unteren zweiten Boden 12b geteilt, wobei sich die Öffnung 6d im oberen zweiten Boden 12a befindet.

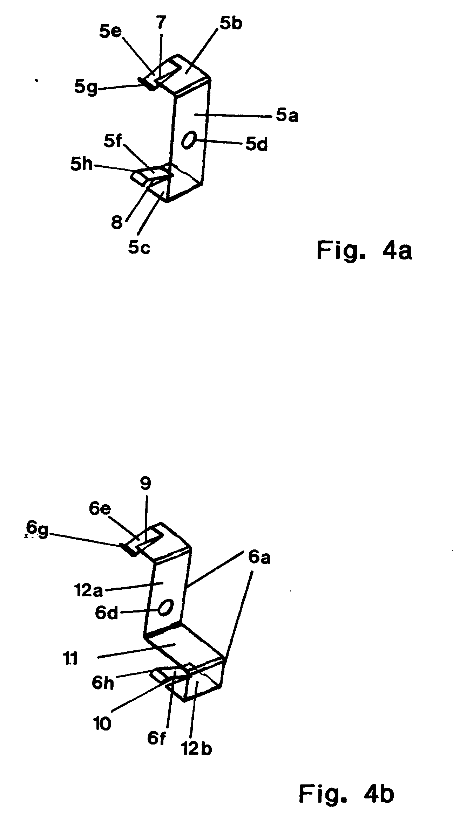

Figur 4a zeigt ein weiteres erstes Federprofil, das etwas komplizierter aufgebaut ist. Es weist einen ersten Boden 5a, eine erste obere Seitenflanke 5b und eine erste untere Seitenflanke 5c auf. Im ersten Boden 5a befindet sich eine Öffnung 5d, über welche das erste Federprofil mit einem Steg 4a, 4b verbunden werden kann. Die erste obere Seitenflanke 5b weist einen Schlitz 7 auf und die erste untere Seitenflanke 5c weist einen Schlitz 8 auf. Jeweils mindestens ein Bereich 5e der ersten oberen Seitenflanke 5b und ein Bereich 5f der ersten unteren Seitenflanke 5c ist ausgehend vom Ende der Schlitze 7, 8 im Hinblick auf die jeweilige Seitenflanke in Richtung der Öffnung 5d angewinkelt. Zudem weist der angewinkelte Bereich 5e, 5f jeweils einen Knick 5g, 5h auf. Dadurch wird das Einschieben eines Strahlungsfilters in die Federprofile erleichtert. In der Rahmenanordnung gemäß Figur 1 oder Figur 2 zeigen die Bereiche 5e, 5f der ersten Federprofile 3a, 3c zueinander. Es ist aber genauso möglich,dass die Bereiche 5e, 5f der ersten Federprofile 3a, 3c voneinander weg oder in die gleiche Richtung zeigen.

Figur 4b zeigt ein weiteres zweites Federprofil, das etwas komplizierter aufgebaut ist. Es weist einen zweiten Boden 6a, eine zweite obere Seitenflanke 6b und eine zweite untere Seitenflanke 6c auf. Im zweiten Boden 6a befindet sich eine Öffnung 6d, über welche das zweite Federprofil mit einem Steg 4a, 4b verbunden werden kann. Die zweite obere Seitenflanke 6b weist einen Schlitz 9 auf und die zweite untere Seitenflanke 6c weist einen Schlitz 10 auf. Jeweils mindestens ein Bereich 6e der zweiten oberen Seitenflanke 6b und ein Bereich 6f der zweiten unteren Seitenflanke 6c ist ausgehend vom Ende der Schlitze 9, 10 im Hinblick auf die jeweilige Seitenflanke in Richtung der Öffnung 6d angewinkelt. Zudem weist der angewinkelte Bereich 6e, 6f jeweils einen Knick 6g, 6h auf. Dadurch wird das Einschieben eines Strahlungsfilters in die Federprofile erleichtert. In der Rahmenanordnung gemäß Figur 1 oder Figur 2 zeigen die Bereiche 6e, 6f der ersten Federprofile 3b, 3d zueinander. Es ist aber genauso möglich,dass die Bereiche 6e, 6f der ersten Federprofile 3b, 3d voneinander weg oder in die gleiche Richtung zeigen.

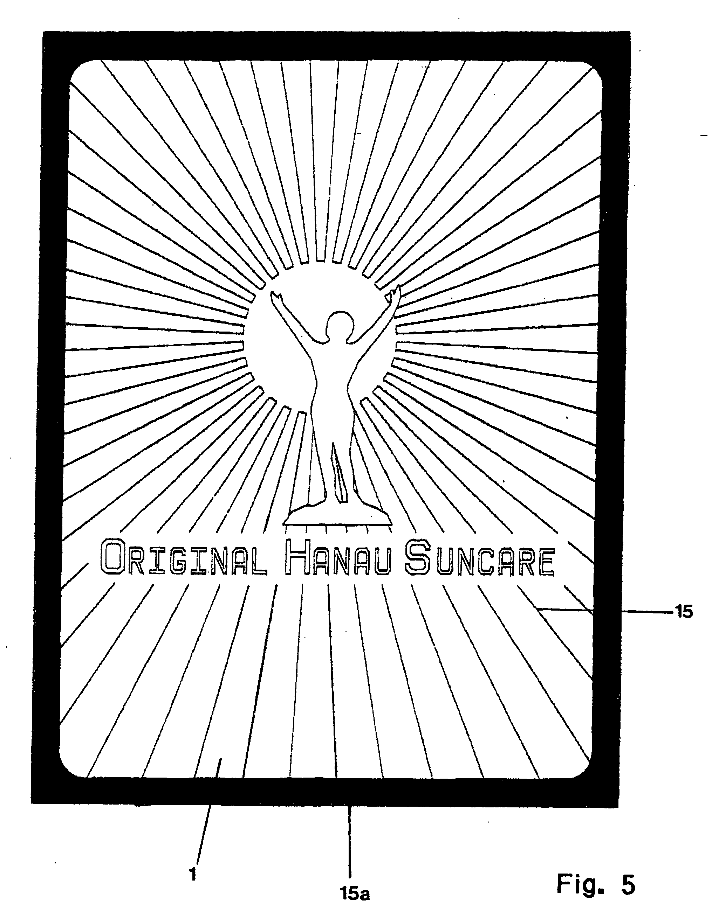

Figur 5 zeigt einen Aufdruck 15 mit undurchsichtigem Randbereich 15a auf dem Strahlungsfilter 1, der die Halterung der Strahlungsfilter vor dem Auge eines Benutzers verdecken soll.

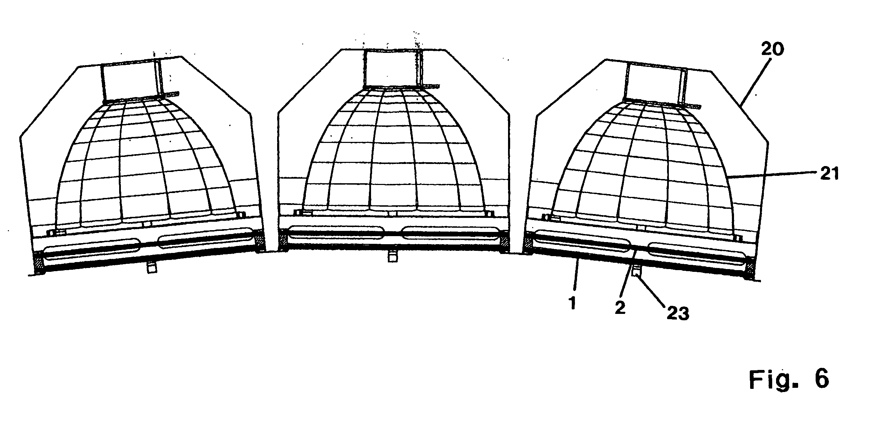

Figur 6 zeigt drei nebeneinander angeordnete Bräunungsmodule im Schnitt, wobei jedes Modul ein Gehäuse 20 und die erfindungsgemäße Rahmenanordnung aufweist. Es sind der Reflektor 21 im Gehäuse 20 und der erste Strahlungsfilter 1 sowie der zweite Strahlungsfilter 2 erkennbar.

Figur 7 zeigt, wie die Rahmenanordnung mit der Verbindungsstange 13 in eine Öffnung 22 im Gehäuse 20 über einen Schwenkmechanismus eingehakt ist. Ein Verschluss 23 fixiert die Rahmenanordnung in ihrer Position ( siehe auch Figur 6 ).

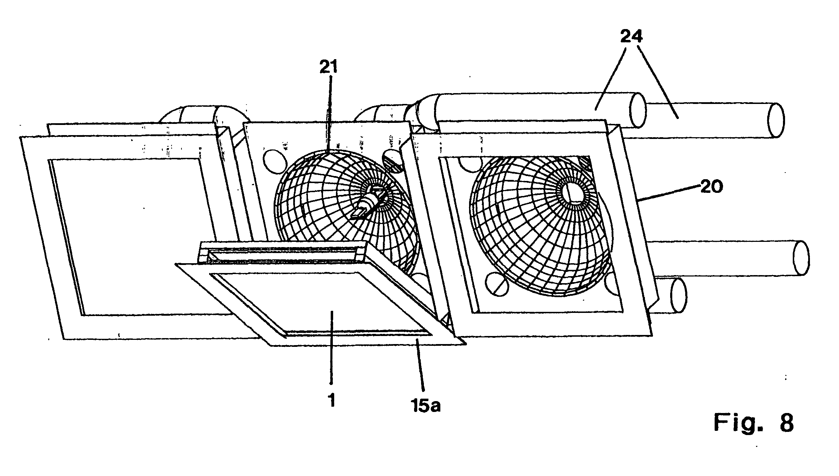

Figur 8 zeigt eine dreidimensionale Darstellung von drei Bräunungsmodulen. Ganz links ist ein Bräunungsmodul mit geschlossener Rahmenanordnung gezeigt. In der Mitte ist ein Bräunungsmodul mit aufgeklappter Rahmenanordnung inklusive Bräunungsstrahler abgebildet, wobei der Reflektor 21, der erste Strahlungsfilter 1 und der undurchsichtige Rahmen 15a erkennbar ist. Ganz rechts ist ein Bräunungsmodul ohne Strahlungsfilter in der Rahmenanordnung und ohne Bräunungsstrahler gezeigt. Alle drei Bräunungsmodule sind mit Luftabsaugschläuchen 24 versehen.

## Patentansprüche

1. Rechteckige Rahmenanordnung mit ein bis zwei scheibenförmigen Strahlungsfiltem zum Abfiltem eines Spektrums eines Bräunungsstrahler, wobei die Rahmenanordnung vier Ecken und vier Seitenkanten aufweist und an einer ersten Seitenkante mindestens ein erstes Federprofil (3a, 3c) und an einer der ersten Seitenkante gegenüberliegenden zweiten Seitenkante mindestens ein zweites Federprofil (3b, 3d) aufweist, wobei zumindest das erste Federprofil (3a, 3c) U-förmig mit einem ersten Boden (5a), einer ersten oberen Seitenflanke (5b) und einer ersten unteren Seitenflanke (5c) ausgestaltet ist, **dadurch gekennzeichnet, dass** das erste Federprofil (3a, 3c) und das zweite Federprofil (3b, 3d) durch mindestens einen Steg (4a, 4b) miteinander verbunden sind und dass ein erster Strahlungsfilter (1) in einem ersten Randbereich zwisehen dem mindestens einen Steg (4a, 4b) und der ersten unteren Seitenflanke (5c) angeordnet ist.

2. Rechteckige Rahmenanordnung nach Anspruch 1, **dadurch gekennzeichnet, dass** das zweite Federprofil (3b, 3d) U-förmig mit einem zweiten Boden (6a), einer zweiten oberen Seitenflanke (6b) und einer zweiten unteren Seitenflanke (6c) ausgestaltet ist.

3. Rechteckige Rahmenanordnung nach einem der Ansprüche 1 bis 2, **dadurch gekennzeichnet, dass** die Winkel zwischen erstem Boden (5a) und erster oberer Seitenflanke (5b) sowie zwischen erstem Boden (5a) und erster unterer Seitenflanke (5c) kleiner 90° sind.

4. Rechteckige Rahmenanordnung nach einem der Ansprüche 2 bis 3, **dadurch gekennzeichnet, dass** die Winkel zwischen zweitem Boden (6a) und zweiter oberer Seitenflanke (6b) sowie zwischen zweitem Boden (6a) und zweiter unterer Seitenflanke (6c) kleiner 90° sind.

5. Rechteckige Rahmenanordnung nach einem der Ansprüche 1 bis 2, **dadurch gekennzeichnet, dass** die Winkel zwischen erstem Boden (5a) und erster oberer Seitenflanke (5b) sowie zwischen erstem Boden (5a) und erster unterer Seitenflanke (5c) gleich 90° sind, dass die erste obere Seitenflanke (5b) und die erste untere Seitenflanke (5c) jeweils mindestens einen Schlitz (7, 8) aufweisen und dass jeweils mindestens ein Bereich (5e) der ersten oberen Seitenflanke (5b) und ein Bereich (5f) der ersten unteren Seitenflanke (5c) ausgehend vom Ende der Schlitze (7, 8) im Hinblick auf die jeweilige Seitenflanke angewinkelt ist.

6. Rechteckige Rahmenanordnung nach einem der Ansprüche 1 bis 2, **dadurch gekennzeichnet, dass** die Winkel zwischen zweitem Boden (6a) und zweiter oberer Seitenflanke (6b) sowie zwischen zweitem Boden (6a) und zweiter unterer Seitenflanke (6c) gleich 90° sind, dass die zweite obere Seitenflanke (6b) und die zweite untere Seitenflanke (6c) jeweils mindestens einen Schlitz (9, 10) aufweisen und dass jeweils mindestens ein Bereich (6e) der zweiten oberen Seitenflanke (6b) und ein Bereich (5f) der zweiten unteren Seitenflanke (6c) ausgehend vom Ende der Schlitze (9, 10) im Hinblick auf die jeweilige Seitenflanke angewinkelt ist.

7. Rechteckige Rahmenanordnung nach einem der Ansprüche 2 oder 6, **dadurch gekennzeichnet, dass** der zweite Boden (6a) durch eine Stufe (11) in einen oberen zweiten Boden (12a) und einen unteren zweiten Boden (12b) geteilt ist.

8. Rechteckige Rahmenanordnung nach Anspruch 7, **dadurch gekennzeichnet, dass** der obere zweite Boden (12a) und der untere zweite Boden (12b) ungleich breit sind.

9. Rechteckige Rahmenanordnung nach einem der Ansprüche 2 bis 3, **dadurch gekennzeichnet, dass** der erste Strahlungsfilter (1) in einem zweiten Randbereich zwischen dem mindestens einen Steg (4a, 4b) und der zweiten unteren Seitenflanke (6c) angeordnet ist.

10. Rechteckige Rahmenanordnung nach einem der Ansprüche 2 bis 9, **dadurch gekennzeichnet, dass** ein zweiter Strahlungsfilter (2) in einem ersten Randbereich zwischen dem mindestens einen Steg (4a, 4b) und der ersten oberen Seitenflanke (5b) und in einem zweiten Randbereich zwischen dem mindestens einen Steg (4a, 4b) und der zweiten oberen Seitenflanke (6b) angeordnet ist.

11. Rechteckige Rahmenanordnung nach einem der Ansprüche 1 bis 10, **dadurch gekennzeichnet, dass** die Federprofile (3a, 3b, 3c, 3d) aus Federblech gebildet sind

12. Rechteckige Rahmenanordnung nach einem der Ansprüche 1 bis 11, **dadurch gekennzeichnet, dass** zwei erste Federprofile (3a, 3c) an der ersten Seitenkante und zwei zweite Federprofile (3b, 3d) an der zweiten Seitenkante angeordnet sind, wobei jeweils ein Steg (4a, 4b) eines der zwei ersten Federprofile (3a, 3c) mit einem der zwei zweiten Federprofile (3b, 3d) verbindet, und wobei die insgesamt vier Federprofile (3a, 3b, 3c, 3d) jeweils nahe einer der vier Ecken der Rahmenanordnung angeordnet sind und die Stege (4a, 4b) über mindestens eine Verbindungsstange (13) miteinander verbunden sind.

13. Rechteckige Rahmenanordnung nach einem der Ansprüche 1 bis 12, **dadurch gekennzeichnet, dass** der erste Strahlungsfilter (1) einen rechteckigen Umfang aufweist.

14. Rechteckige Rahmenanordnung nach einem der Ansprüche 1 bis 13, **dadurch gekennzeichnet, dass** der erste Strahlungsfilter (1) ein Interferenzfilter ist.

15. Rechteckige Rahmenanordnung nach einem der Ansprüche 13 bis 14, **dadurch gekennzeichnet, dass** der erste Strahlungsfilter (1) eine Breite und eine Länge im Bereich von 210mm bis 300mm aufweist.

16. Rechteckige Rahmenanordnung nach Anspruch 15, **dadurch gekennzeichnet, dass** der erste Strahlungsfilter (1) eine Breite von 220mm und eine Länge von 290mm aufweist.

17. Rechteckige Rahmenanordnung nach einem der Ansprüche 10 bis 16, **dadurch gekennzeichnet, dass** der zweite Strahlungsfilter (2) ein UV-Filter oder ein Infrarotfilter ist.

18. Rechteckige Rahmenanordnung nach einem der Ansprüche 10 bis 17, **dadurch gekennzeichnet, dass** der zweite Strahlungsfilter (2) rechteckig ausgebildet ist.

19. Rechteckige Rahmenanordnung nach Anspruch 18, **dadurch gekennzeichnet, dass** der zweite Strahlungsfilter 82) eine Breite und eine Länge im Bereich von 210mm bis 290mm aufweist.

20. Rechteckige Rahmenanordnung nach Anspruch 19, **dadurch gekennzeichnet, dass** der zweite Strahlungsfilter (2) eine Breite von 220mm und eine Länge von 278mm aufweist

21. Rechteckige Rahmenanordnung nach einem der Ansprüche 1 bis 20, **dadurch gekennzeichnet, dass** der Steg (4a, 4b) auf halber Höhe zwischen der ersten oberen Seitenflanke (5b) und der ersten unteren Seitenflanke (5c) mit dem ersten Boden (5a) verbunden ist.

22. Rechteckige Rahmenanordnung nach einem der Ansprüche 1 bis 6, **dadurch gekennzeichnet, dass** der Steg (4a, 4b) auf halber Höhe zwischen der zweiten oberen Seitenflanke (6b) und der zweiten unteren-Seitenflanke (6c) mit dem zweiten Boden (6a) verbunden ist.

23. Rechteckige Rahmenanordnung nach einem der Ansprüche 7 bis 22, **dadurch gekennzeichnet, dass** der Steg (4a, 4b) zwischen der zweiten oberen Seitenflanke (6b) und der Stufe (11) mit dem oberen zweiten Boden (12a) verbunden ist.

24. Rechteckige Rahmenanordnung nach einem der Ansprüche 7 bis 23, **dadurch gekennzeichnet, dass** der Steg (4a, 4b) zwischen der zweiten unteren Seitenflanke (6c) und der Stufe (11) mit dem unteren zweiten Boden (12b) verbunden ist.

25. Rechteckige Rahmenanordnung nach einem der Ansprüche 1 bis 24, **dadurch gekennzeichnet, dass** die oberen Seitenflanken (5b, 6b) und/oder die unteren Seitenflanken (5c, 6c) einen Knick (5g, 5f, 6g, 6f) aufweisen.

26. Rechteckige Rahmenanordnung nach einem der Ansprüche 1 bis 25, **dadurch gekennzeichnet, dass** an den Seitenkanten der Rahmenanordnung, an denen kein Federprofil vorhanden ist, jeweils eine Rutschsicherung für den ersten Strahlungsfilter (1) angeordnet ist.

27. Rechteckige Rahmenanordnung nach einem der Ansprüche 10 bis 26, **dadurch gekennzeichnet, dass** an den Seitenkanten der Rahmenanordnung, an denen kein Federprofil vorhanden ist, jeweils eine Rutschsicherung für den zweiten Strahlungsfilter (2) angeordnet ist.

28. Rechteckige Rahmenanordnung nach einem der Ansprüche 1 bis 27, **dadurch gekennzeichnet, dass** der erste Strahlungsfilter (1) auf seiner dem zweiten Strahlungsfilter (2) abgewandten Seite einen Aufdruck oder Aufkleber (15) aufweist.

29. Rechteckige Rahmenanordnung nach Anspruch 28, **dadurch gekennzeichnet, dass** der Aufdruck oder Aufkleber (15) einen undurchsichtigen Randbereich (15a) aufweist.

30. Bräunungsmodul mit einem Gehäuse (20), einem im oder am Gehäuse (20) angeordneten, dreidimensionalen Reflektor (21) sowie einer rechteckigen Rahmenanordnung nach einem der Ansprüche 1 bis 29 an einer Seite des Gehäuses (20), wobei der erste Strahlungsfilter (1) die Strahlungsaustrittsfläche des Reflektors (21) überdeckt, wobei die obere erste Seitenflanke (5b) und die obere zweite Seitenflanke (6b) des Federprofils (3a, 3b, 3c, 3d) dem Reflektor (21) zugewandt sind, während die untere erste Seitenflanke (5c) und die untere zweite Seitenflanke (6c) des Federprofils (3a, 3b, 3c, 3d) vom Reflektor (21) abgewandt sind.

31. Bräunungsmodul nach Anspruch 30, **dadurch gekennzeichnet, dass** die rechteckige Rahmenanordnung über einen Schwenkmechanismus vom Gehäuse (20) lösbar ist.

32. Bräunungsmodul nach Anspruch 31, **dadurch gekennzeichnet, dass** die rechteckige Rahmenanordnung in das Gehäuse (20) eingehakt ist.

33. Bräunungsmodul nach Anspruch 31, **dadurch gekennzeichnet, dass** die rechteckige Rahmenanordnung in eine Öffnung (22) gemäß Figur 7 im Gehäuse (20) eingehakt ist.

34. Bräunungsmodul nach einem der Ansprüche 28 bis 31, **dadurch gekennzeichnet, dass** die rechteckige Rahmenanordnung mittels eines Schnappmechanismus (23) in der Position fixiert wird.

35. Bräunungsmodul nach einem der Ansprüche 30 bis 34, **dadurch gekennzeichnet, dass** ein Umfang des Reflektors (21) parallel zur Strahlungsaustrittsfläche einen Kreis, eine Ellipse, ein Rechteck oder ein Vieleck beschreibt.

36. Bräunungsmodul nach Anspruch 35, **dadurch gekennzeichnet, dass** der Reflektor (21) aus Facetten gebildet ist und der Umfang des Reflektors (21) parallel zur Strahlungsaustrittsfläche ein Vieleck mit zwölf Ecken beschreibt.

37. Bräunungsmodul nach Anspruch 36, **dadurch gekennzeichnet, dass** der Reflektor (21) eine Höhe von 90mm bis 95mm, insbesondere von 93,6mm, und das Zwölfeck in der Ebene der Strahlungsaustrittsfläche einen maximalen Durchmesser ( von Ecke zu Ecke ) im Bereich von 210mm bis 230mm, insbesondere von 210mm, aufweist.

38. Bräunungsmodul nach Anspruch 36, **dadurch gekennzeichnet, dass** der Reflektor (21) eine Höhe im Bereich von 110mm bis 125mm, insbesondere von 118,7mm, und das Zwölfeck in der Ebene der Strahlungsaustrittsfläche einen maximalen Durchmesser ( von Ecke zu Ecke ) im Bereich von 170mm bis 200mm, insbesondere von 184mm, aufweist.

39. Bräunungsmodul nach Anspruch 36, **dadurch gekennzeichnet, dass** der Reflektor (21) eine Höhe im Bereich von 75mm bis 90mm, insbesondere von 83,3mm, und das Zwölfeck in der Ebene der Strahlungsaustrittsfläche einen maximalen Durchmesser ( von Ecke zu Ecke ) im Bereich von 205mm bis 235mm, insbesondere von 220mm, aufweist.

## Claims

1. Rectangular frame arrangement with one to two disc-shaped radiation filters for filtering out a spectrum of a tanning radiator, wherein the frame arrangement has four corners and four lateral edges and, on a first lateral edge, has at least a first spring profile (3a, 3c) and on a second lateral edge opposite the first lateral edge, has at least a second spring profile (3b, 3d), wherein at least the first spring profile (3a, 3c) is U-shaped with a first base (5a), a first upper lateral flank (5b) and a first lower lateral flank (5c), **characterised in that** the first spring profile (3a, 3c) and the second spring profile (3b, 3d) are connected to one another by at least one web (4a, 4b) and **in that** a first radiation filter (1) is arranged in a first marginal region between the at least one web (4a, 4b) and the first lower lateral flank (5c).

2. Rectangular frame arrangement according to claim 1, **characterised in that** the second spring profile (3b, 3d) is U-shaped with a second base (6a), a second upper lateral flank (6b) and a second lower lateral flank (6c).

3. Rectangular frame arrangement according to either of claims 1 or 2, **characterised in that** the angles between the first base (5a) and the first upper lateral flank (5b) and between the first base (5a) and the first lower lateral flank (5c) are less than 90°.

4. Rectangular frame arrangement according to either of claims 2 or 3, **characterised in that** the angles between the second base (6a) and second upper lateral flank (6b) and between the second base (6a) and second lower lateral flank (6c) are less than 90°.

5. Rectangular frame arrangement according to either of claims 1 or 2, **characterised in that** the angles between the first base (5a) and first upper lateral flank (5b) and between the first base (5a) and first lower lateral flank (5c) are equal to 90°, **in that** the first upper lateral flank (5b) and the first lower lateral flank (5c) each have at least one slot (7, 8) and that at least one respective region (5e) of the first upper lateral flank (5b) and one region (5f) of the first lower lateral flank (5c) are bent starting from the end of the slot (7, 8) with regard to the respective lateral flank.

6. Rectangular frame arrangement according to either of claims 1 or 2, **characterised in that** the angles between the second base (6a) and second upper lateral flank (6b) and between the second base (6a) and the second lower lateral flank (6c) are equal to 90°, **in that** the second upper lateral flank (6b) and the second lower lateral flank (6c) each have at least one slot (9, 10) and that at least one respective region (6e) of the second upper lateral flank (6b) and a region (5f) of the second lower lateral flank (6c) are bent starting from the end of the slot (9, 10) with regard to the respective lateral flank.

7. Rectangular frame arrangement according to either of claims 2 or 6, **characterised in that** the second base (6a) is divided by a step (11) into an upper second base (12a) and a lower second base (12b).

8. Rectangular frame arrangement according to claim 7, **characterised in that** the upper second base (12a) and the lower second base (12b) are of unequal width.

9. Rectangular frame arrangement according to either of claims 2 or 3, **characterised in that** the first radiation filter (1) is arranged in a second marginal region between the at least one web (4a, 4b) and the second lower lateral flank (6c).

10. Rectangular frame arrangement according to any one of claims 2 to 9, **characterised in that** a second radiation filter (2) is arranged in a first marginal region between the at least one web (4a, 4b) and the first upper lateral flank (5b) and in a second marginal region between the at least one web (4a, 4b) and the second upper lateral flank (6b).

11. Rectangular frame arrangement according to any one of claims 1 to 10, **characterised in that** the spring profiles (3a, 3b, 3c, 3d) are formed from spring steel.

12. Rectangular frame arrangement according to any one of claims 1 to 11, **characterised in that** two first spring profiles (3a, 3c) are arranged on the first lateral edge and two second spring profiles (3b, 3d) are arranged on the second lateral edge, wherein one respective web (4a, 4b) connects one of the two first spring profiles (3a, 3c) to one of the two second spring profiles (3b, 3d), and wherein the total of four spring profiles (3a, 3b, 3c, 3d) are each arranged close to one of the four comers of the frame arrangement and the webs (4a, 4b) are connected to one another via at least one connecting rod (13).

13. Rectangular frame arrangement according to any one of claims 1 to 12, **characterised in that** the first radiation filter (1) has a rectangular periphery.

14. Rectangular frame arrangement according to any one of claims 1 to 13, **characterised in that** the first radiation filter (1) is an interference filter.

15. Rectangular frame arrangement according to either of claims 13 to 14, **characterised in that** the first radiation filter (1) has a width and a length in the range 210mm to 300mm.

16. Rectangular frame arrangement according to claim 15, **characterised in that** the first radiation filter (1) has a width of 220mm and a length of 290mm.

17. Rectangular frame arrangement according to any of claims 10 to 16, **characterised in that** the second radiation filter (2) is an UV filter or an infrared filter.

18. Rectangular frame arrangement according to any one of claims 10 to 17, **characterised in that** the second radiation filter (2) is rectangular.

19. Rectangular frame arrangement according to claim 18, **characterised in that** the second radiation filter (2) has a width and a length in the range 210mm to 290mm.

20. Rectangular frame arrangement according to claim 19, **characterised in that** the second radiation fitter (2) has a width of 220mm and a length of 278mm.

21. Rectangular frame arrangement according to any one of claims 1 to 20, **characterised in that** the web (4a, 4b) is connected to the first base (5a) halfway up between the first upper lateral flank (5b) and the first lower lateral flank (5c).

22. Rectangular frame arrangement according to any one of claims 1 to 6, **characterised in that** the web (4a, 4b) is connected to the second base (6a) halfway up between the second upper lateral flank (6b) and the second lower lateral flank (6c).

23. Rectangular frame arrangement according to any one of claims 7 to 22, **characterised in that** the web (4a, 4b) is connected to the upper second base (12a) between the second upper lateral flank (6b) and the step (11).

24. Rectangular frame arrangement according to any one of claims 7 to 23, **characterised in that** the web (4a, 4b) is connected to the lower second base (12b) between the second lower lateral flank (6c) and the step (11).

25. Rectangular frame arrangement according to any one of claims 1 to 24, **characterised in that** the upper lateral flanks (5b, 6b) and/or the lower lateral flanks (5c, 6c) have a bend (5g, 5f, 6g, 6f).

26. Rectangular frame arrangement according to any one of claims 1 to 25, **characterised in that** a respective anti-slip device for the first radiation filter (1) is arranged on the lateral edges of the frame arrangement, on which no spring profile is present,

27. Rectangular frame arrangement according to any one of claims 10 to 26, **characterised in that** a respective anti-slip device for the second radiation filter (2) is arranged on the lateral edges of the frame arrangement, on which no spring profile is present.

28. Rectangular frame arrangement according to any one of claims 1 to 27, **characterised in that** the first radiation filter (1) has an imprint or adhesive label (15) on its side remote from the second radiation filter (2).

29. Rectangular frame arrangement according to claim 28, **characterised in that** the imprint or adhesive label (15) has an opaque marginal region (15a).

30. Tanning module comprising a housing (20), a three-dimensional reflector (21) arranged in or on the housing (20) and a rectangular frame arrangement according to any of claims 1 to 29 on a side of the housing (20), wherein the first radiation filter (1) overlaps the radiation exit area of the reflector (21), the upper first lateral flank (5b) and the upper second lateral flank (6b) of the spring profile (3a, 3b, 3c, 3d) facing the reflector (21), while the lower first lateral flank (5c) and the lower second lateral flank (6c) of the spring profile (3a, 3b, 3c, 3d) are remote from the reflector (21).

31. Tanning module according to claim 30, **characterised in that** the rectangular frame arrangement can be detached from the housing (20) via a pivoting mechanism.

32. Tanning module according to claim 31, **characterised in** the rectangular frame arrangement is hooked into the housing (20).

33. Tanning module according to claim 31, **characterised in that** the rectangular frame arrangement is hooked into an opening (22) in the housing (20) according to Figure 7.

34. Tanning module according to any one of claims 28 to 31, **characterised in that** the rectangular frame arrangement is fixed in position by means of a snap mechanism (23).

35. Tanning module according to any one of claims 30 to 34, **characterised in that** a periphery of the reflector (21) parallel to the radiation exit area describes a circle, an ellipse, a rectangle or a polygon.

36. Tanning module according to claim 35, **characterised in that** the reflector (21) is formed by facets and the periphery of the reflector (21) parallel to the radiation exit area describes a polygon with twelve comers.

37. Tanning module according to claim 36, **characterised in that** the reflector (21) has a height of 90mm to 95mm, in particular 93.6mm, and the dodecagon in the plane of the radiation exit area has a maximum diameter (from corner to comer) in the range 210mm to 230mm, in particular 210mm.

38. Tanning module according to claim 36, **characterised in that** the reflector (21) has a height in the range 110mm to 125mm, in particular 118.7mm, and the dodecagon in the plane of the radiation exit area has a maximum diameter (from corner to comer) in the range 170mm to 200mm, in particular 184mm.

39. Tanning module according to claim 36, **characterised in that** the reflector (21) has a height in the range 75mm to 90mm, in particular 83.3mm, and the dodecagon in the plane of the radiation exit area has a maximum diameter (from comer to comer) in the range 205mm to 235mm, in particular 220mm.

## Revendications

1. Agencement de cadre rectangulaire comportant un ou deux filtres de rayonnement en forme de plaques servant à filtrer un spectre d'une lampe de bronzage, l'agencement de cadre présentant quatre coins et quatre arêtes latérales, et au moins un premier profil-ressort (3a, 3c) sur une première arête latérale et au moins un deuxième profil-ressort (3b, 3d) sur une deuxième arête latérale opposée à la première arête latérale, au moins le premier profil (3a, 3c) est configuré en forme de U avec un premier fond (5a), un premier flanc latéral supérieur (5b) et un premier flanc latéral inférieur (5c), **caractérisé en ce que** le premier profil-ressort (3a, 3c) et le deuxième profil-ressort (3b, 3d) sont reliés l'un à l'autre par au moins une traverse (4a, 4b), et **en ce qu'**un premier filtre de rayonnement (1) est agencé dans une première région de bord entre ladite au moins une traverse (4a, 4b) et le premier flanc latéral inférieur (5c).

2. Agencement de cadre rectangulaire selon la revendication 1, **caractérisé en ce que** le deuxième profil-ressort (3b, 3d) est configuré en forme de U avec un deuxième fond (6a), un deuxième flanc latéral supérieur (6b) et un deuxième flanc latéral inférieur (6c).

3. Agencement de cadre rectangulaire selon l'une des revendications 1 à 2, **caractérisé en ce que** les angles entre le premier fond (5a) et le premier flanc latéral supérieur (5b) ainsi qu'entre le premier fond (5a) et le premier flanc latéral inférieur (5c) sont inférieurs à 90°.

4. Agencement de cadre rectangulaire selon l'une des revendications 2 à 3, **caractérisé en ce que** les angles entre le deuxième fond (6a) et le deuxième flanc latéral supérieur (6b) ainsi qu'entre le deuxième fond (6a) et le deuxième flanc latéral inférieur (6c) sont inférieurs à 90°.

5. Agencement de cadre rectangulaire selon l'une des revendications 1 à 2, **caractérisé en ce que** les angles entre le premier fond (5a) et le premier flanc latéral supérieur (5b) ainsi qu'entre le premier fond (5a) et le premier flanc latéral inférieur (5c) sont égaux à 90°, **en ce que** le premier flanc latéral supérieur (5b) et le premier flanc latéral inférieur (5c) présentent chacun au moins une fente (7, 8), et **en ce qu'**au moins une région (5e) du premier flanc latéral supérieur (5b) et une région (5f) du premier flanc latéral inférieur (5c) sont chacune coudées en partant de l'extrémité des fentes (7, 8) par rapport au flanc latéral respectif.

6. Agencement de cadre rectangulaire selon l'une des revendications 1 à 2, **caractérisé en ce que** les angles entre le deuxième fond (6a) et le deuxième flanc latéral supérieur (6b) ainsi qu'entre le deuxième fond (6a) et le deuxième flanc latéral inférieur (6c) sont égaux à 90°, **en ce que** le deuxième flanc latéral supérieur (6b) et le deuxième flanc latéral inférieur (6c) présentent chacun au moins une fente (9, 10), et **en ce qu'**au moins une région (6e) du deuxième flanc latéral supérieur (6b) et une région (6f) du deuxième flanc latéral inférieur (6c) sont chacune coudées en partant de l'extrémité des fentes (9, 10) par rapport au flanc latéral respectif.

7. Agencement de cadre rectangulaire selon l'une ou l'autre des revendications 2 et 6, **caractérisé en ce que** le deuxième fond (6a) est divisé par un gradin (11) en un deuxième fond supérieur (12a) et en un deuxième fond inférieur (12b).

8. Agencement de cadre rectangulaire selon la revendication 7, **caractérisé en ce que** le deuxième fond supérieur (12a) et le deuxième fond inférieur (12b) sont de largeur inégale.

9. Agencement de cadre rectangulaire selon l'une des revendications 2 à 3, **caractérisé en ce que** le premier filtre de rayonnement (1) est agencé dans une deuxième région de bord entre ladite au moins une traverse (4a, 4b) et le deuxième flanc latéral inférieur (6c).

10. Agencement de cadre rectangulaire selon l'une des revendications 2 à 9, **caractérisé en ce qu'**un deuxième filtre de rayonnement (2) est agencé dans une première région de bord entre ladite au moins une traverse (4a, 4b) et le premier flanc latéral supérieur (5b) et dans une deuxième région de bord entre ladite au moins une traverse (4a, 4b) et le deuxième flanc latéral supérieur (6b).

11. Agencement de cadre rectangulaire selon l'une des revendications 1 à 10, **caractérisé en ce que** les profils-ressorts (3a, 3b, 3c, 3d) sont formés en tôle à ressort.

12. Agencement de cadre rectangulaire selon l'une des revendications 1 à 11, **caractérisé en ce que** deux premiers profils-ressorts (3a, 3c) sont agencés sur la première arête latérale et deux deuxièmes profils-ressorts (3b, 3d) sont agencés sur la deuxième arête latérale, une traverse respective (4a, 4b) reliant l'un des deux premiers profils-ressorts (3a, 3c) à l'un des deux deuxièmes profils-ressorts (3b, 3d), et tous les quatre profils-ressorts (3a, 3b, 3c, 3d) étant agencés chacun à proximité d'un des quatre coins de l'agencement de cadre, et les traverses (4a, 4b) étant reliées l'une à l'autre par au moins une tige de liaison (13).

13. Agencement de cadre rectangulaire selon l'une des revendications 1 à 12, **caractérisé en ce que** le premier filtre de rayonnement (1) présente une périphérie rectangulaire.

14. Agencement de cadre rectangulaire selon l'une des revendications 1 à 13, **caractérisé en ce que** le premier filtre de rayonnement (1) est un filtre d'interférence.

15. Agencement de cadre rectangulaire selon l'une des revendications 13 à 14, **caractérisé en ce que** le premier filtre de rayonnement (1) présente une largeur et une longueur dans la plage de 210 mm à 300 mm.

16. Agencement de cadre rectangulaire selon la revendication 15, **caractérisé en ce que** le premier filtre de rayonnement (1) présente une largeur de 220 mm et une longueur de 290 mm.

17. Agencement de cadre rectangulaire selon l'une des revendications 10 à 16, **caractérisé en ce que** le deuxième filtre de rayonnement (2) est un filtre UV ou un filtre à infrarouge.

18. Agencement de cadre rectangulaire selon l'une des revendications 10 à 17, **caractérisé en ce que** le deuxième filtre de rayonnement (2) est réalisé rectangulaire.

19. Agencement de cadre rectangulaire selon la revendication 18, **caractérisé en ce que** le deuxième filtre de rayonnement (2) présente une largeur et une longueur dans la plage de 210 mm à 290 mm.

20. Agencement de cadre rectangulaire selon la revendication 19, **caractérisé en ce que** le deuxième filtre de rayonnement (2) présente une largeur de 220 mm et une longueur de 278 mm.

21. Agencement de cadre rectangulaire selon l'une des revendications 1 à 20, **caractérisé en ce que** la traverse (4a, 4b) est reliée avec le premier fond (5a) à mi-hauteur entre le premier flanc latéral supérieur (5b) et le premier flanc latéral inférieur (5c).

22. Agencement de cadre rectangulaire selon l'une des revendications 1 à 6, **caractérisé en ce que** la traverse (4a, 4b) est reliée au deuxième fond (6a) à mi-hauteur entre le deuxième flanc latéral supérieur (6b) et le deuxième flanc latéral inférieur (6c).

23. Agencement de cadre rectangulaire selon l'une des revendications 7 à 22, **caractérisé en ce que** la traverse (4a, 4b) est reliée au deuxième fond supérieur (12a) entre le deuxième flanc latéral supérieur (6b) et le gradin (11).

24. Agencement de cadre rectangulaire selon l'une des revendications 7 à 23, **caractérisé en ce que** la traverse (4a, 4b) est reliée au deuxième fond inférieur (12b) entre le deuxième flanc latéral inférieur (6c) et le gradin (11).

25. Agencement de cadre rectangulaire selon l'une des revendications 1 à 24, **caractérisé en ce que** les flancs latéraux supérieurs (5b, 6b) et/ou les flancs latéraux inférieurs (5c, 6c) présentent une flexion (5g, 5f, 6g, 6f).

26. Agencement de cadre rectangulaire selon l'une des revendications 1 à 25, **caractérisé en ce que** sur les arêtes latérales de l'agencement de cadre rectangulaire, sur lesquelles il n'y a pas de profil-ressort, est agencé un dispositif antidérapant pour le premier filtre de rayonnement (1).

27. Agencement de cadre rectangulaire selon l'une des revendications 10 à 26, **caractérisé en ce que** sur les arêtes latérales de l'agencement de cadre rectangulaire, sur lesquelles il n'y a pas de profil-ressort, est agencé un dispositif antidérapant pour le deuxième filtre de rayonnement (2).

28. Agencement de cadre rectangulaire selon l'une des revendications 1 à 27, **caractérisé en ce que** le premier filtre de rayonnement (1) présente sur sa face détournée du deuxième filtre de rayonnement (2) une impression ou un autocollant (15).

29. Agencement de cadre rectangulaire selon la revendication 28, **caractérisé en ce que** l'impression ou l'autocollant (15) présente une région de bord (15a) non transparente.

30. Module de bronzage comportant un boîtier (20), un réflecteur (21) tridimensionnel agencé dans ou sur le boîtier (20), ainsi qu'un agencement de cadre rectangulaire selon l'une des revendications 1 à 29 sur un côté du boîtier (20), le premier filtre de rayonnement (1) recouvrant la surface de sortie de rayonnement du réflecteur (21), le premier flanc latéral supérieur (5b) et le deuxième flanc latéral supérieur (6b) du profil-ressort (3a, 3b, 3c, 3d) étant tournés vers le réflecteur (21) tandis que le premier flanc latéral inférieur (5c) et le deuxième flanc latéral inférieur (6c) du profil-ressort (3a, 3b, 3c, 3d) sont détournés du réflecteur (21).

31. Module de bronzage selon la revendication 30, **caractérisé en ce que** l'agencement de cadre rectangulaire peut être détaché du boîtier (20) via un mécanisme de pivotement.

32. Module de bronzage selon la revendication 31, **caractérisé en ce que** l'agencement de cadre rectangulaire est accroché dans le boîtier (20).

33. Module de bronzage selon la revendication 31, **caractérisé en ce que** l'agencement de cadre rectangulaire est accroché dans le boîtier (20) dans une ouverture (22) selon la figure 7.

34. Module de bronzage selon l'une des revendications 28 à 31, **caractérisé en ce que** l'agencement de cadre rectangulaire est fixé dans la position au moyen d'un mécanisme d'encliquetage (23).

35. Module de bronzage selon l'une des revendications 30 à 34, **caractérisé en ce qu'**une périphérie du réflecteur (21) décrit parallèlement à la surface de sortie de rayonnement un cercle, une ellipse, un rectangle ou un polygone.

36. Module de bronzage selon la revendication 35, **caractérisé en ce que** le réflecteur (21) est formé par des facettes, et la périphérie du réflecteur (21) décrit parallèlement à la surface de sortie de rayonnement un polygone à douze coins.

37. Module de bronzage selon la revendication 36, **caractérisé en ce que** le réflecteur (21) présente une hauteur de 90 mm à 95 mm, en particulier de 93,6 mm, et le dodécagone présente dans le plan de la surface de sortie de rayonnement un diamètre maximum (de coin à coin) dans la plage de 210 mm à 230 mm, en particulier de 210 mm.

38. Module de bronzage selon la revendication 36, **caractérisé en ce que** le réflecteur (21) présente une hauteur de 110 mm à 125 mm, en particulier de 118,7 mm, et le dodécagone présente dans le plan de la surface de sortie de rayonnement un diamètre maximum (de coin à coin) dans la plage de 170 mm à 200 mm, en particulier de 184 mm.

39. Module de bronzage selon la revendication 36, **caractérisé en ce que** le réflecteur (21) présente une hauteur de 75 mm à 90 mm, en particulier de 83,3 mm, et le dodécagone présente dans le plan de la surface de sortie de rayonnement un diamètre maximum (de coin à coin) dans la plage de 205 mm à 235 mm, en particulier de 220 mm.
